# EUROPEAN PATENT APPLICATION

(11) **EP 0 640 318 A1**
(43) Date of publication of application: **01.03.1995**
(21) Application number: 94830310.2
(22) Date of filing: 23.06.1994
(51) Int. Cl.: A61B 17/58

(54) **Endomedullar nail for reducing proximal femur fractures and apparatus for locating the nail**

(30) Priority: 30.08.1993 IT MI931864
(71) Applicant: SYNOS MEDICAL S.p.A., I-20156 Milano (IT)
(72) Inventor: Spotorno, Lorenzo, Synos Medical S.p.A, I-20156 Milano (IT); Bergadano, Dario, Synos Medical S.p.A, I-20156 Milano (IT)
(74) Representative: Cicogna, Franco

(57) **Abstract**

The present invention relates to an endomedullar nail for reducing the proximal femur fractures, which nail comprises an insert or infibulum element (1) of elongated shape, which can be introduced into the femur bone and has an elliptical section at the proximal region.

The infibulum is provided, in its proximal portion, with two holes (4,5) which are parallel to one another and slanted with respect to the axis of the proximal portion, for introducing a stabilizing cervical screw (10) and a bearing cervical screw (11) having a diameter larger than that of the stabilizing cervical screw.

## Description

### BACKGROUND OF THE INVENTION

The present invention relates to an endomedullar nail for reducing the proximal femur fractures, and also relates to an apparatus for locating said nail.

As is known, in the case of a fracture of the proximal portion of the femur bone, it is frequently necessary or anyhow advisable to adopt reducing or synthesis means allowing to hold the fracture portions at a mutually suitable position and, moreover, for properly transmitting the loads through the traumatized region.

The synthesis or reducing means which are at present used conventionally comprise outer fixing elements, metal plates to be connected to bone screws, and insert or infibulum elements to be arranged inside the medullar channel.

With respect to prior insert or infibulum elements, they are affected by great coupling problems, because of the difficulty of properly locking against rotation the infibulum with respect to the femur bone, while holding the infibulum properly arranged with respect to the bone.

### SUMMARY OF THE INVENTION

Accordingly, the aim of the present invention is to overcome the above mentioned drawbacks, by providing an endomedullar nail which can be firmly anchored in the involved bone region, while preventing radial compression stresses from being transmitted to the femur bone.

Within the scope of the above mentioned aim, a main object of the present invention is to provide such an endomedullar nail which can properly stabilize a diaphisary region of the femur, with respect to the proximal region thereof.

Another object of the present invention is to provide such an endomedullar nail which is also adapted to prevent rotary movements as well as other displacements of the femur bone portions to be reunited.

Yet another object of the present invention is to provide an apparatus which allows to apply the endomedullar nail to the femur bone with a very great accuracy, and also affords the possibility of properly applying all of the stabilizing means.

According to one aspect of the present invention, the above mentioned aim and objects, as well as yet other objects, which will become more apparent hereinafter, are achieved by an endomedullar nail for reducing the proximal femur fractures, characterized in that said nail comprises an infibulum which can be inserted into the medullar channel of the femur bone and having an elliptical section at the proximal region, said infibulum being further provided, at the proximal portion thereof, with two parallel holes which are slanted with respect to the axis of the proximal portion, for engaging therein a stabilizing cervical screw and a bearing cervical screw respectively.

### BRIEF DESCRIPTION OF THE DRAWINGS

Further characteristics and advantages of the present invention will become more apparent hereinafter from the following detailed disclosure of the preferred, though not exclusive, embodiment of an endomedullar nail for reducing proximal femur fractures, and of the apparatus for locating or installing said nail, which are illustrated, by way of an indicative, but not limitative, example, in the figures of the accompanying drawings, where:

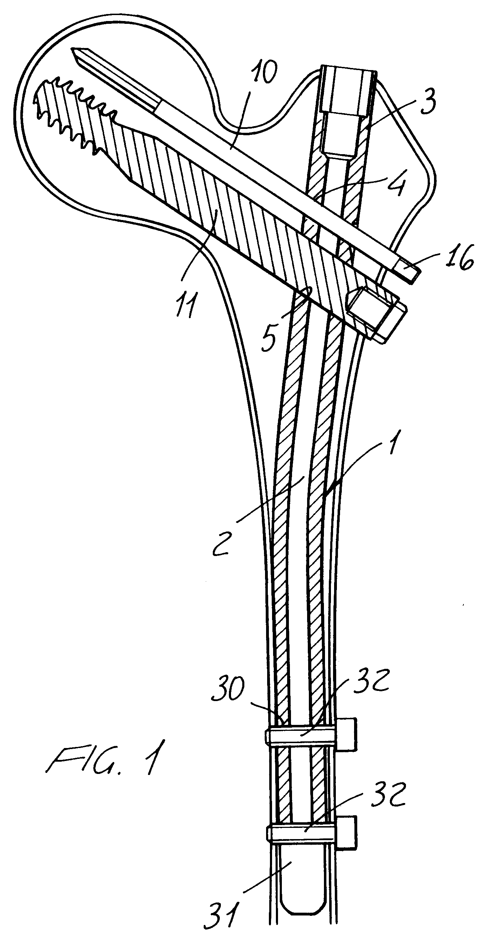

Figure 1 is a schematic view illustrating the endomedullar nail according to the present invention arranged in a femur bone;
Figure 2 illustrates the endomedullar nail and the apparatus for its application;

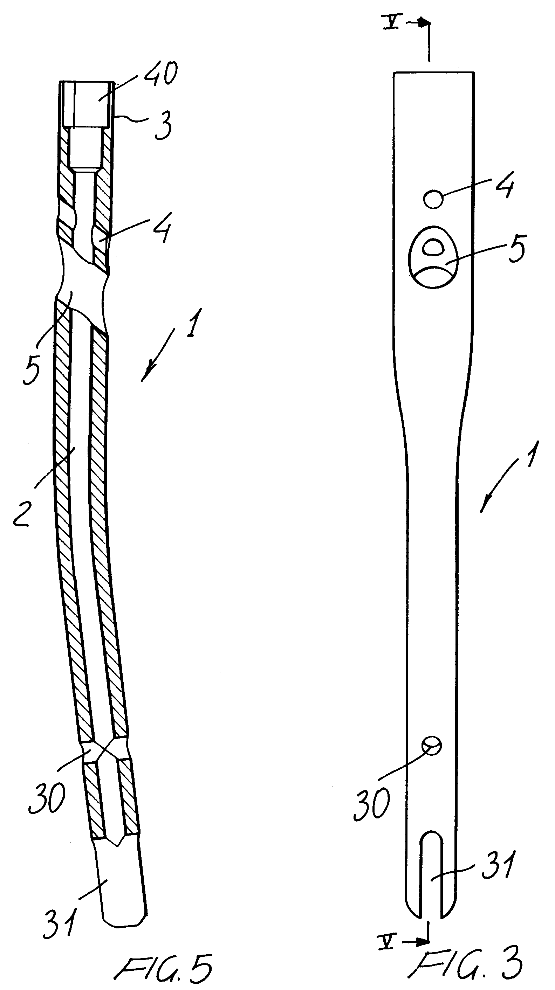

Figure 3 is a front view illustrating the subject endomedullar nail;

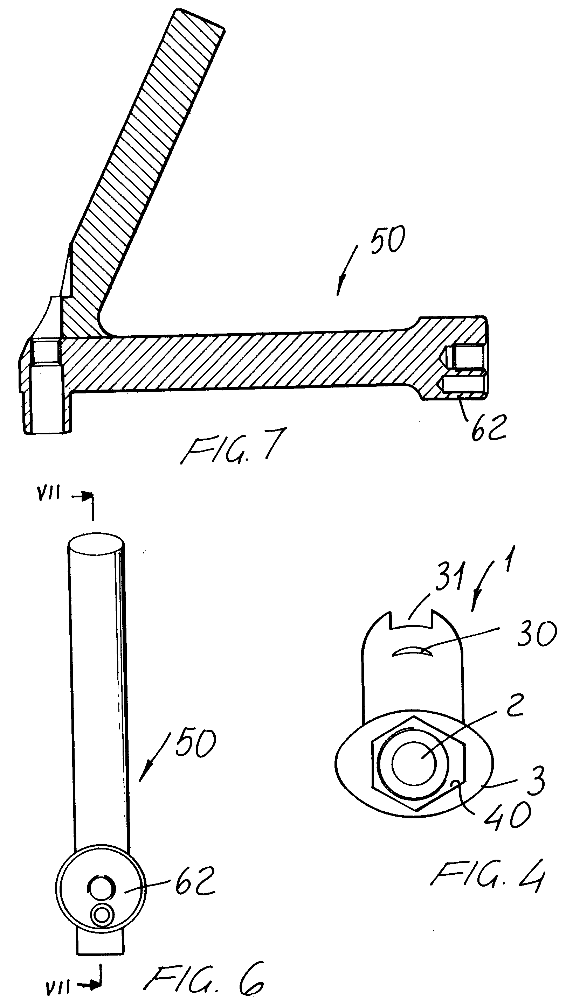

Figure 4 is a side end view illustrating the subject endomedullar nail;

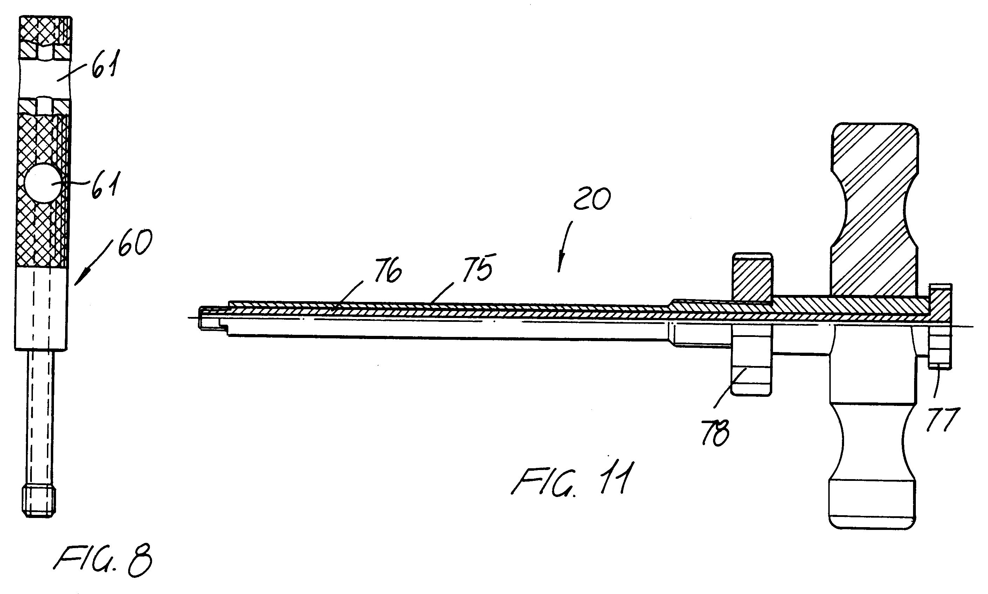

Figure 5 is a cross-sectional view substantially taken along the line V-V of Figure 3;
Figure 6 is a front view illustrating a handle element for locating the endomedullar line;
Figure 7 is a cross-sectional view substantially taken along the line VII-VII of Figure 6;
Figure 8 is a partial cross-sectional view illustrating the affixing cannulated screw;
Figure 9 is a front view illustrating a guide element;

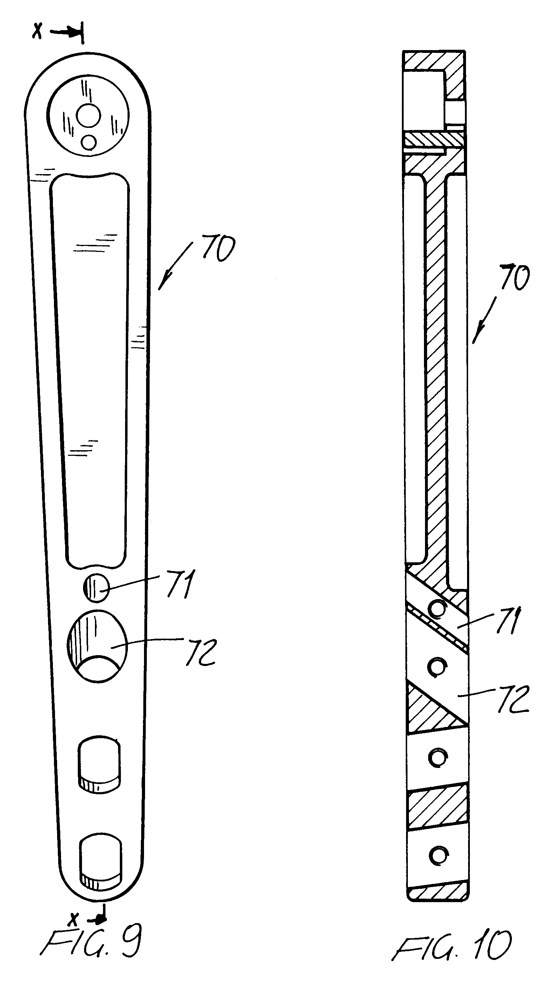

Figure 10 is a cross-sectional view substantially taken along the line X-X of Figure 9;
   and
Figure 11 is a partially cross-sectioned view illustrating a screw-driver element for applying the bearing cervical screw.

### DESCRIPTION OF THE PREFERRED EMBODIMENT

With reference to the number references of the above mentioned figures, the endomedullar nail for reducing proximal femur fractures, according to the present invention, comprises generally an insert or infibulum element, generally indicated at the reference number 1, which is provided with an axial channel 2 extending for the overall length thereof, so as to provide the infibulum with a closed section which does not subject the femur bone to any radial compression stresses.

The infibulum 1, at the proximal end portion thereof, has an elliptical section 3, which is adapted to be housed in a preliminarily prepared recess in the proximal region of the femur bone, and which operates for preventing any rotations about the axis of the proximal region of the nail from occurring, while preventing the generation of shear effects which would hinder a proper bone regrowth.

At the proximal region of the infibulum there are provided a first and second holes 4 and 5 which have parallel axes and are slanted with respect to the axis of the proximal portion.

Moreover, the second hole 5 has advantageously a diameter larger than the diameter of the first hole 4.

In the first hole 4, in particular, is engaged a stabilizing cervical screw 10, whereas in the second hole 5, of larger diameter, there is engaged a bearing cervical screw 11.

The parallel relationship of the axis of the two cervical screws will allow a possible sliding of the bone in the parallel direction, which feature will be useful in the case in which the screws traverse the fracture line.

More specifically, the stabilizing cervical screw 10 is provided for holding in a proper position the neck and head of the femur bone, during the forming of the hole and the introduction of the bearing cervical screw and will prevent the femur neck from rotating as the device is used.

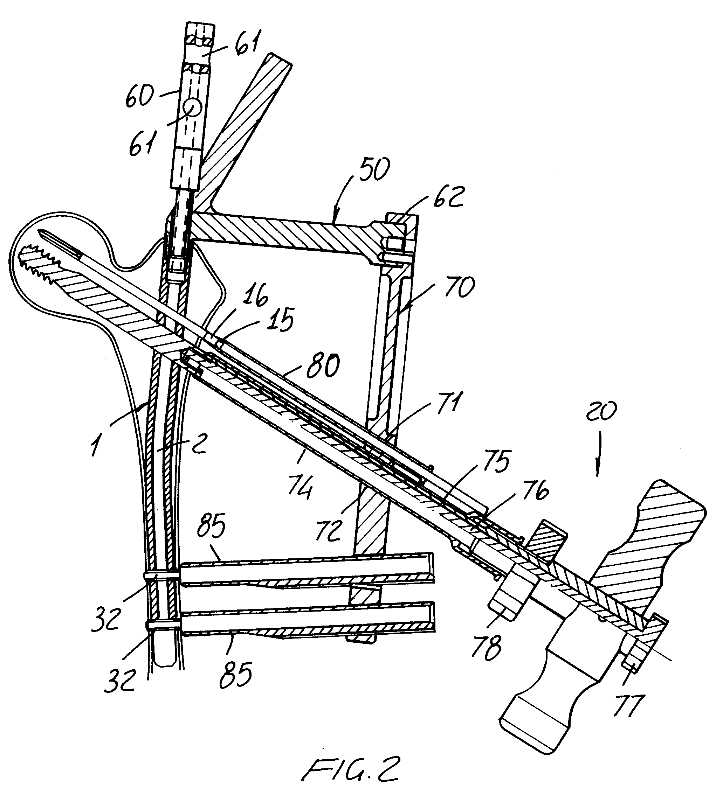

The smaller diameter cervical screw 10 is provided, at an intermediate portion thereof, and as is clearly shown in Figure 2, with a tapering portion 15 adjoining a threaded portion 16 so as to provide a preset breaking point, thereon the operator will apply a force in order to cut the screw at a set length.

The leftward thread 16 will allow to screw-in a suitable tool, for removing the screw upon consolidation of the fracture.

In order to actuate the cervical screw 10, there is provided a screw-driver element, generally indicated at the reference number 20, which will be disclosed in a more detailed manner hereinafter.

At the distal end portion, the infibulum is provided with a throughgoing hole and a cut-out or notch 31 arranged at the free end portion thereof.

In the throughgoing hole and notch can be engaged additional screws 32 operating for stabilizing the diaphisary region of the femur, with respect to the proximal region thereof.

The notch 31, provided at the distal end portion of the nail, has the function of allowing a bone screw to be dynamically assembled, that is it will allow the nail body to distally slide.

The selection of a static or a dynamic assembling will be selected by the operator, during the operation, and will be afforded by a suitable mechanism provided on the perforating guide.

Moreover, the distal stiffness of the nail is thereby reduced, so as to allow the nail to have a performance like that of the bone.

At the proximal end portion of the nail and parallel to the axis thereof, there is provided a seat or recess 40 having the shape of an uneven hexagon, which engages with a corresponding projecting portion of a handle 50, or key, so as to affix the mutual position of the infibulum and operating key or handle, while assuring a strong and accurate connection.

In particular, the operating key or handle is made rigid with the infibulum by means of a knurled and cannulated screw 60, having cross holes 61, for housing therein metal rods to be used for withdrawing the nail.

Alternatively, an impact mass system can be affixed to the threaded end portion XX (see Figure 7) of the operating key or handle 50, for allowing the nail to be easily withdrawn.

The handle key, moreover, is provided with a centering seat 52 in which there is engaged a locating guide 70 therethrough there are formed throughgoing holes for applying the cervical screws and distal screws.

In particular, the locating element 70 is provided with a first parallel-axis holes 71 and 72, in which there is engaged a sleeve element for the application of the stabilizing cervical screw.

In the hole 72 there is engaged a first tubular guide 74, in the inside of which is engaged a second tubular guide 75, in which can be engaged the screw-driver 20.

The second tubular guide 75 will allow to properly guide a Kirschner wire, which is introduced so as to nearly contact the cortical portion of the femur bone.

Into the second tubular guide 74, on the other hand, there is introduced the mentioned special screw- driver 20, provided with threaded stem 76 which, as operated by the operator through the outer wheel 77, will engage with the bearing cervical screw 10 and will allow to provide a pulling force owing to the provision of an outer nut 78 which will affect the end portion of the first tubular guide.

In order to apply the endomedullar nail according to the present invention, the cortical portion of the femur is at first perforated at the greater trochanter; then, a suitable guide wire will be introduced through the made opening.

The honing of the channel is performed by means of a cannulated milling element and a flexible shaft and through a suitable rasping element there is formed an ellipsoidal recess where will be housed the proximal end portion of the infibulum which is assembled to the operating handle by means of the connection screw 60; then, the guide element 70 will be affixed to the handle 50.

The infibulum will be introduced into the cavity formed in the femur bone and will be brought to its end position.

After having inserted the infibulum, the small diameter cervical screw will be applied, through a tubular guide 80, engaged in the hole 71 defined on the guide element 70.

After having applied the tubular guide there is engaged the screw 11, through a conventional Jacobs mandrel or, depending on the requirements, by means of a suitable screw-driver.

The screw, which is of a self-perforating and self-threading type, is introduced so as to cause the tapered portion, forming the breaking point, to be arranged at about 8-10 mm from the outer cortical portion of the femur.

After having introduced this screw in the disclosed manner, there is performed the cutting or shearing operation.

In order to introduce the bearing cervical screw, the first tubular guide is introduced into the provided seat of the angled guide and on the cortical portion there is made a hole; then the second tubular guide is introduced into the first and, after having performed the milling of the inside of the bone, the bearing cervical screw is introduced, by the screw-driver 20 to the desired position.

In the case in which a pulling force must be applied to the screw, is operated the nut 78, which is manually driven by the operator.

In the case in which is necessary to apply distal screws, there are used special tubular guides, indicated at 85, which are engaged in corresponding holes formed on the locating element 70.

The holes in the bone are performed with a conventional surgical drill.

From the above disclosure it should be apparent that the invention fully achieves the intended aim and objects.

In particular, the fact is to be pointed out that an endomedullar nail has been provided which is particularly firmly affixed inside the bone and allow to obtain a better bone regrowth condition.

The invention as disclosed is susceptible to several modifications and variations all of which will come within the scope of the inventive idea.

Moreover, all of the details can be replaced by other technically equivalent elements.

In practicing the invention, the used materials, provided that they are compatible to the intended use, as well as the contingent size and shapes, can be any, depending on requirements.

## Claims

1. An endomedullar nail for reducing the proximal femur fractures, characterized in that said nail comprises an infibulum which can be inserted into the medullar channel of the femur bone and having an elliptical section at the proximal region, said infibulum being further provided, at the proximal portion thereof, with two parallel holes which are slanted with respect to the axis of the proximal portion, for engaging therein a stabilizing cervical screw and a bearing cervical screw respectively.

2. An endomedullar nail, according to the preceding claim, characterized in that said bearing cervical screw has a diameter larger than the diameter of said stabilizing cervical screw.

3. An endomedullar nail, according to the preceding claims, characterized in that said infibulum is provided with an axial recess extending for the overall length of said infibulum.

4. An endomedullar nail, according to one or more of the preceding claims, characterized in that said infibulum, at the distal end portion thereof, is provided with a throughgoing hole as well as with a fork portion.

5. An endomedullar nail, according to one or more of the preceding claims, characterized in that said infibulum is provided, at the proximal end portion thereof, with an uneven polygonal seat which can be coupled to an operating handle element.

6. An apparatus for locating an endomedullar nail according to the preceding claims, characterized in that said apparatus comprises an operating handle to be engaged in an uneven polygonal seat defined at the proximal end portion of an infibulum, and being moreover provided an affixing cannulated screw which can be engaged in said seat, said operating handle defining a contoured end portion which can be engaged in the seat of a guide element defining throughgoing slanted holes, parallel to one another, for housing therein tubular guides and for engaging therein distal screws.

7. An apparatus, according to Claim 6, characterized in that said apparatus further comprises a screw-driver, engageable in a tubular guide, and provided with a threaded stem which can be coupled with said bearing cervical screw and connected to an operating wheel for operating said bearing cervical screw, there being moreover provided a pulling nut, which is rotatably supported by said screw-driver and abutting on said first tubular guide.

8. An apparatus, according to Claims 6 and 7, characterized in that said stabilizing cervical screw is provided, at an intermediate portion thereof, with a tapering portion adjoining a threaded portion, said tapering portion being adapted to be arranged near a cortical portion of the femural bone head in order to cut the screw to provide a thread for the connection with means for withdrawing the screws upon consolidation of the bone fracture.
